# EUROPEAN PATENT SPECIFICATION

(11) **EP 2 351 534 B1**
(45) Date of publication and mention of the grant of the patent: **22.08.2012**
(21) Application number: 10305103.3
(22) Date of filing: 01.02.2010
(51) Int. Cl.: A61B 17/70

(54) **Intervertebral device**
Zwischenwirbelvorrichtung
Dispositif intervertébral

(43) Date of publication of application: 03.08.2011
(73) Proprietor: ZIMMER SPINE, 33000 Bordeaux (FR)
(72) Inventor: Douget, Stéphane, 33110, LE BOUSCAT (FR)
(74) Representative: Besnard, Christophe Laurent

(56) References cited:
- EP-A1- 2 138 122
- FR-A1- 2 921 248
- US-A- 6 099 527
- US-A1- 2009 018 662

## Description

### FIELD OF THE INVENTION

The present disclosure relates to an intervertebral device to be placed between two spinous processes of two vertebrae. This device is also often called "interspinous device" or "interspinous implant".

Such a device is typically used for holding two vertebrae in a desired relative position while allowing, in some cases, a limited amount of relative movement between these two vertebrae.

### BACKGROUND OF THE INVENTION

The spine is formed of superposed vertebrae, from the lumbar vertebrae to the cervical vertebrae, each having an anterior part, which is the vertebral body, and a posterior part, which is the vertebral arch (or neural arch), the anterior and posterior parts enclosing the vertebral foramen. Each vertebral arch is formed by a pair of pedicles and a pair of laminae, and has transverse processes and/or a spinous process (or neural spine) projecting therefrom. The transverse and spinous processes project opposite to the vertebral foramen.

Intervertebral discs lie between each pair of adjacent vertebrae (i.e. between the vertebral bodies of these vertebrae). Each disc forms a cartilaginous joint to hold the two adjacent vertebrae together while allowing slight relative movements between these vertebrae.

When an intervertebral disk has worn out or has degenerated, it becomes unable to prevent excessive movements between the two vertebrae which surround the disk, during flexion (forward movement) or extension (backward movement) of the spine. As a result, the anterior or posterior parts of the vertebrae come too close together and may even come into contact with each other in the worst cases, which causes discomfort and/or pain to the patient. More particularly, when the posterior parts of the vertebrae come too close together, the spinal nerves may be pinched between the vertebrae, which is very painful.

As a remedy to this problem, it is known to place an intervertebral device between the spinous processes of the two adjacent vertebrae. Such a device compensates for the deficiency of the disk, especially by limiting the extent to which the posterior parts of the two vertebrae can move towards each other when the spine is extended.

A type of device known in the art comprises a spacer having two opposite faces, each face being provided with a groove adapted to receive a spinous process, and one or two ties being adapted to surround the spinous processes and being fixed to the spacer. Due to the spacer, the vertebrae are prevented from coming too close together and, due to the tie(s), they are retained in said grooves and prevented from moving apart too much.

Known examples of intervertebral device of the above type are disclosed, for instance, in US patents n° 7087083 B2, n° 7163558 B2 and n° 7520887 B2.

Such known devices comprise a spacer with an elbow-shaped inner passage opening into two adjacent outer faces of the spacer, and a tie having proximal and distal portions both fixed to the spacer. The proximal portion of the tie is assembled to the spacer as follows: the end of the proximal portion is passed through said elbow-shaped inner passage, and then the proximal portion is folded back and sewed on itself. These assembling steps require one to thoroughly handle the tie and the spacer, for a significant time. Moreover, the assembling requires one to sew the tie while it is already preassembled to the spacer. So, the sewing step is made difficult because of the spacer.

Other examples of intervertebral devices are disclosed in published patent applications n° FR 2 921 248 and EP 2 138 122 A1.

There is a need for an intervertebral device that is easier to assemble.

### SUMMARY OF THE INVENTION

According to one embodiment of the present disclosure, there is provided an intervertebral device to be placed between two spinous processes of two vertebrae, as defined in appended claim 1. Such a device comprises: a spacer having two opposite faces, each being adapted to engage a spinous process, and at least one elongated member for maintaining the engagement of the faces to the spinous processes, said elongated member having a proximal portion; wherein the spacer is provided with at least one set of first and second holes, the first hole being adapted to receive said proximal portion which is to be inserted into the first hole in a first direction, and the second hole intersecting the first hole; wherein the device further comprises a fixing member moveable between an unlocked position and a locked position, the fixing member going through the second hole and protruding into the first hole in said locked position; and wherein said proximal portion is adapted for interacting with said fixing member so as to impede relative translation movement between the proximal portion and the spacer, in the first direction, when the fixing member is in its locked position.

Therefore, the elongated member may be easily assembled to the spacer by means of the fixing member. Compared to the devices of the prior art, the proposed device is easier to assemble, especially because there is no more need to thoroughly handle the spacer or to use a sewing machine while tying the proximal portion of the elongated member to the spacer.

Such a spacer may be used, for instance, between the spinous processes of two lumbar vertebrae, or between the spinous processes of the first sacral vertebra (called S1) and the fifth lumbar vertebra (called L5).

According to an embodiment, the proximal portion of the elongated member forms a loop, the fixing member (more precisely, the part of the fixing member protruding into the first hole) passing through said loop in said locked position.

Said loop may be formed in many ways, including by folding back and fixing the proximal portion to itself, e.g. by sewing, gluing or welding. In any case, the loop forming step is done before assembling the proximal portion to the spacer. For instance, it may be done while manufacturing the elongated member.

According to an embodiment, the second hole extends in a second direction which is substantially orthogonal to the first direction.

According to an embodiment, the second hole opens into a side face of the spacer.

According to an embodiment, the fixing member has first and second parts and an intermediate part therebetween, wherein the first and second parts are substantially straight and the intermediate part is elbow-shaped, the first part going through the second hole of the spacer and the second part resting on an outer face of the spacer when the fixing member is in its locked position.

Thus, the fixing member is easily locked by pushing it into the second hole, until the second part of the fixing member comes into abutment on said outer face of the spacer.

According to an embodiment, the spacer is provided with a notch on its outer face, this notch extending from the second hole in a third direction which is substantially orthogonal to the second direction, and being adapted to receive the second part of the fixing member.

Said notch impedes relative rotation movement between the fixing member and the spacer, i.e. it impedes the rotation of the fixing member around said second direction, by jamming the second part of the fixing member. Moreover, the depth of the notch is usually higher than the thickness of the second part, so that the second part is better jammed and does not protrude over the outer face of the spacer.

According to an embodiment, said notch extends in a third direction which is substantially orthogonal to the first direction.

Thus, when the fixing member is in its locked position, the forces exerted on the fixing member by the elongated member, which are mainly oriented in the first direction, have as less effect as possible on the behavior of the second part of the fixing member which is located in the notch.

According to an embodiment, the spacer comprises at least one finger or lip extending over said notch and being configured for retaining the second part of the fixing member in said notch, thereby allowing one to clip the fixing member into the notch. The clip-fixing of the fixing member onto the spacer reduces the risk of loosing the fixing member and makes the device easier to assemble and safer to use.

According to an embodiment, the spacer further comprises at least one fixing system for fixing a distal portion of the elongated member to the spacer. Said fixing system may be, for instance, a clip-fixing system, a clamping system, a self-locking system or a combination thereof.

The elongated member may be made from a deformable material that allows a certain amount of movement so that, even after the physician (or other operator) has pulled and locked in position the proximal and distal portions of the elongated member, the elongated member allows a limited amount of relative movement between the vertebrae while providing a stabilizing effect. The elongated member may be made from a polymeric material such as, for example, polyester, polyethylene (for example, polyethylene terephthalate, i.e. PET), polyetheretherketone (PEEK) or any other material that provides the desired deformability and flexibility. The elongated member may be a tie having a band shape, a cord shape or other shapes. For example, it may be made by weaving.

The spacer may be made, for example, of polyetheretherketone (PEEK) or titanium alloy.

The central part of the spacer may be slightly deformable or not. When the central part is slightly deformable, it allows a limited amount of relative movement between the vertebrae. Especially, the central part of the spacer may be slightly deformable in compression so as to allow the posterior parts of the vertebrae to move towards each other when the spine is extended.

According to an embodiment, the opposite faces of the spacer each further comprise a groove adapted to receive a spinous process.

According to an embodiment, at least one of said two opposite faces comprises a groove defined between two flanges, and said first hole goes through one of said flanges and opens into the face of the spacer. Thus, the proximal portion of the elongated member which is inserted in the first hole extends substantially in line with said flange, which improves the holding of the spinous process.

### BRIEF DESCRIPTION OF THE DRAWINGS

In the drawings, like reference signs generally refer to the same parts throughout the different views. Moreover, parts or elements of different embodiments having the same or analogous function are identified by the same reference number.

The drawings are not necessarily to scale, emphasis instead being placed upon illustrating the principles of the invention.
FIGS 1 to 3 are perspective views of an example of intervertebral device comprising a fixing member and a tie which are shown in different positions, from one figure (FIG) to the other, from an unlocked position to a locked position.
FIG 4 is a diagrammatical view showing the intervertebral device of FIGS 1 to 3 in place between two adjacent spinous processes.
FIG 5 is a diagrammatical view, as that of FIG 4, showing another example of intervertebral device.

### DETAILED DESCRIPTION OF THE INVENTION

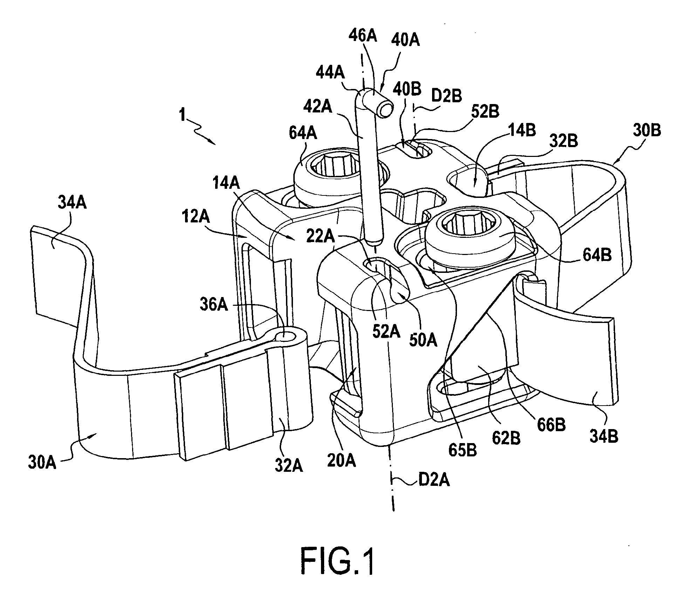

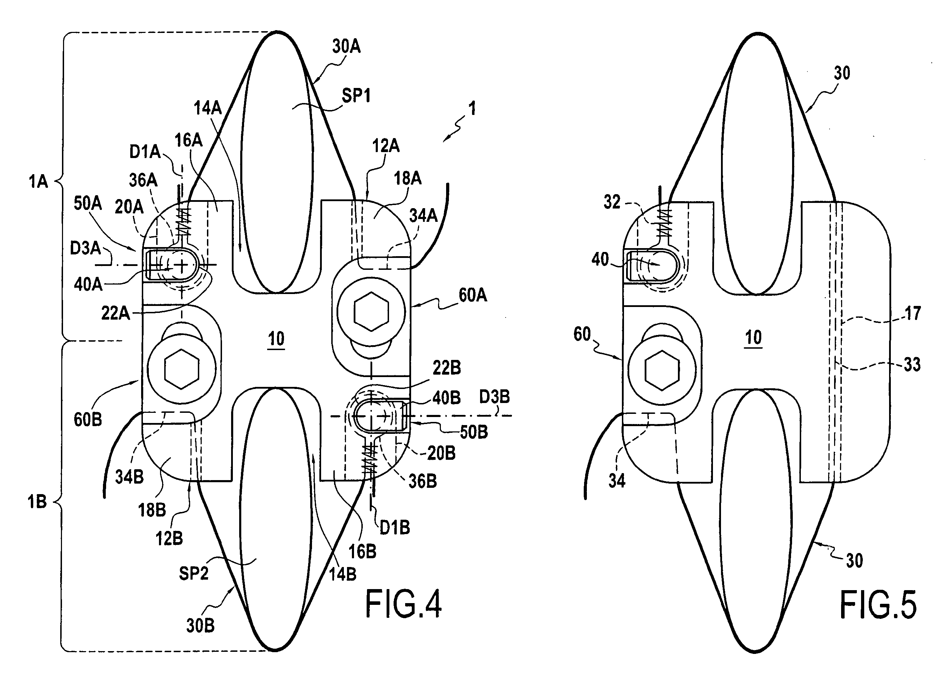

FIGS 1 to 4 show an example of intervertebral device 1 according to the present disclosure, which is adapted to be placed between two adjacent spinous processes SP1, SP2 of two vertebrae, as shown in FIG 4.

This device 1 comprises a spacer 10 having two opposite end faces 12A, 12B, each being adapted to engage one spinous process. These end faces 12A, 12B, are the upper and lower faces of the spacer 10 when it is implanted between two vertebrae, as shown in FIG 4. Each end face 12A (12B) of the spacer is provided with a groove 14A (14B) adapted to receive one of the two spinous processes SP1 (SP2), this groove 14A (14B) being defined between two flanges 16A, 18A (16B, 18B).

As can be seen from FIGS 1 to 4, the upper and lower parts 1A, 1B of the device 1 are analogous. Thus, the subparts or elements of the upper and lower parts 1A, 1B, which are identical or have analogous functions, are identified by the same reference number followed by "A" for the upper part and "B" for the lower part.

The device 1 comprises two ties 30A, 30B having a band shape, each tie 30A (30B) having a proximal portion 32A (32B) and a distal portion 34A (34B). The proximal portion 32A (32B) of each tie 30A (30B) forms a loop 36A (36B). In this example, the ties 30A, 30B are the same. The ties 30A, 30B constitute elongated members according to the present disclosure, the spinous processes SP1, SP2 being retained by the ties 30A, 30B in the grooves 14A, 14B.

The spacer 10 is also provided with two first holes 20A, 20B and two second holes 22A, 22B.

Each first hole 20A (20B) goes through one flange 16A (16B) and opens into one end face 12A (12B) of the spacer 10. It is adapted to receive the proximal portion 32A (32B) of one tie 30A (30B), said proximal portion 32A (32B) being inserted into the first hole 20A (20B) in a first direction D1A (D1B) - see FIG 4.

Each second hole 22A (22B) goes through one flange 16A (16B) and opens into one side face of the spacer 10. It is adapted to receive a fixing member. Each second hole 22A (22B) intersects one first hole 20A (20B) and extends in a second direction D2A (D2B) which is substantially orthogonal to the first direction D1A (D1B).

The device 1 further comprises fixing members, such as pins 40A, 40B which are attachable to the spacer 10. In this example, the fixing pins 40A, 40B are the same. Each fixing pin 40A (40B) has first and second parts 42A, 46A and an intermediate part 44A therebetween. In this example, the first and second parts 42A, 46A are straight, the second part 46A being shorter than the first part 42A, and the intermediate part 44A is elbow-shaped, so that the fixing pin 40A has substantially an L-shape. Each fixing pin 40A (40B) is moveable between an unlocked position and a locked position and, in the locked position, it goes through one second hole 22A (22B) and protrudes into one first hole 20A (20B) of the spacer 10.

The spacer 10 is also provided with notches 50A, 50B on its outer side faces. Each notch 50A (50B) extends from one second hole 22A (22B) in a third direction D3A (D3B) which is substantially orthogonal to both the first and second directions D1A, D2A (D1B, D2B). Each notch 50A (50B) is adapted to receive the second part 44A (44B) of one of said fixing pins 40A (40B). The spacer 10 further comprises a lip 52A (52B) extending over each notch 50A (50B).

The device 1 further comprises two fixing systems 60A, 60B for fixing the distal portion 34A, 34B of the ties 30A, 30B to the spacer 10. In this example, the fixing systems 60A, 60B, are the same.

In a general way, each fixing system 60B (60A) comprises a compression member 62B - see FIG 1 - which is movable relative to the spacer 10. The compression member 62B and the spacer 10 both define clamping surfaces between which distal portion 34B of the tie 30B can be inserted, said distal portion 34B being clamped between said clamping surfaces by moving the compression member 62B relative to the spacer 10.

In the example of FIGS 1 to 4, each fixing system 60B (60A) comprises a screw 64B (64A) with a head and a shaft, the screw shaft having a thread for engagement with another thread provided in the compression member 62B. The compression member 62B is located in a cavity of the spacer 10, this cavity being delimited by an inclined cavity wall 66B. The screw head bears on an outer side face of the spacer 10, and the screw shaft goes through an oblong hole 65B provided in the spacer and communicating with said cavity. By rotating the screw head relative to the spacer 10, the compression member 62B slides on the inclined cavity wall 66B, thereby moving closer or farther apart from another cavity wall. The distal portion 34B of the tie 30B is passed though a slot communicating with said cavity and between the compression member 62B and said other cavity wall.

FIGS 1 to 4 show one example of fixing system but of course, other kinds of fixing system may be used.

Now that the structure of the intervertebral device has been described, the operation of the device is going to be described with reference to FIGS 1 to 3.

FIGS 1 to 3 show how to attach the tie 30A to the spacer 10, the tie 30B being already attached to the spacer 10. In FIG 1, both the proximal and distal portions 32A, 34A of the tie 30A are free.

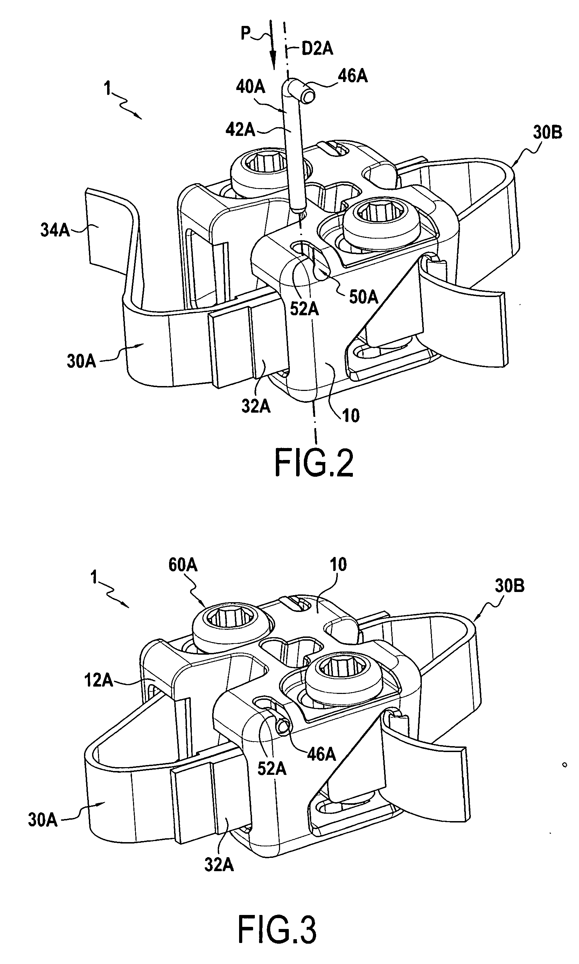

Firstly, the proximal portion 32A is inserted into the first hole 20A in the first direction D1A, so that the loop 36A enters into the first hole 20A up to the bottom of this hole ― see FIG 4. In this position, shown in FIG 2, the loop 36A is in line with the second hole 22A, which means that the second direction D2A goes through the loop 36A.

Then, the fixing pin 40A is pushed into the second hole 22A, as illustrated by arrow P in FIG 2, so that the first part 42A of the fixing pin goes through the second hole 22A and protrudes into the first hole 20A, while the second part 46A is clipped into the notch 50A. More precisely, the second part 46A is inserted by force into the notch 50A, so that the lip 52A is slightly deformed by the pressure exerted by the second part 46A and let the second part 46A enter into the notch 50A. Once the second part 46A has gone beyond the lip 52A, said pressure is no longer applied to the lip 52A and the lip returns to its original shape, thereby enclosing the second part 46A in the notch 50A. Thus, the second part 46A is retained in the notch 50A by the lip 52A, as shown in FIG 3.

When the fixing pin 40A is in its locked position, as shown in FIG 3, the first part 42A, which protrudes into the first hole 20A, goes through the loops 36A of the tie 30A, thereby impeding relative translation movement between the proximal portion 32A of the tie and the spacer 10, in the first direction D1A.

Secondly, the distal portion 34A of the tie is attached to the spacer 10 by means of the fixing system 60A.

Typically, the proximal portions 32A, 32B of the ties 30A, 30B are preassembled to the spacer 10 before packaging the device. Usually, the device is packaged in a sterilized container (e.g. a bag) under an aseptic condition. The packaged device 1 is then stored and/or delivered to a physician, or another operator.

In operative conditions, the device 1 may be used as follows:
- the surgeon creates posterior access to spinous processes SP1, SP2 through an incision in the patient;
- the surgeon inserts the device 1 between the spinous processes SP1, SP2;
- the ties 30A, 30B are passed, respectively, around the spinous processes SP2, SP1, and the distal portions 34A, 34B of said ties are passed, respectively, through the fixing systems 60A, 60B;
- tension is applied to the ties 30A, 30B by pulling, respectively, on the ends of the distal portions 34A, 34B; and
- the distal portions 34A, 34B are, respectively, fixed to the spacer 10 by means of the fixing systems 60A, 60B.

Another example of intervertebral device is shown in FIG 5. This example differs from that of FIGS 1 to 3 in that it comprises one tie 30 (i.e. one elongated member) being adapted to surround the two spinous processes SP1, SP2.

The proximal portion 32 of this tie 30 is fixed to the spacer 10 by means of a fixing pin 40, whereas its distal portion 34 is fixed to the spacer 10 by means of a fixing system 60. The fixing pin 40 and the fixing system are located on one side (i.e. the left side in FIG 5) of the spacer 10. On the opposite side of the spacer 10 (i.e. the right side in FIG 5), a slot 17 is provided for guiding an intermediate portion 33 of the tie 30. Said slot 17 goes through the spacer 10 in this example, but it may be provided on the outer side face of the spacer 10.

The ways to fixe the proximal and distal portions 32, 34 of the tie 30 to the spacer 10 are the same as for the ties 30A, 30B of FIGS 1 to 4.

## Claims

1. An intervertebral device to be placed between two spinous processes of two vertebrae, comprising:
a spacer (10) having two opposite faces (12A, 12B), each face being adapted to engage a spinous process (SP1, SP2), and
at least one elongated member (30A, 30B) for maintaining the engagement of the faces (12A, 12B) to the spinous processes (SP1, SP2), said elongated member having a proximal portion (32A, 32B),
wherein the spacer (10) is provided with at least one set of first and second holes (20A, 20B; 22A, 22B), the first hole (20A, 20B) being adapted to receive said proximal portion (32A, 32B) which is to be inserted into the first hole in a first direction (D1A, D1B), and
wherein the device (1) further comprises a fixing member (40A, 40B) moveable between an unlocked position and a locked position,
the intervertebral device being **characterized in that**
the second hole (22A, 22B) intersects the first hole,
the fixing member (40A, 40B) goes through the second hole (22A, 22B) and protrudes into the first hole (20A, 20B) in said locked position, and
said proximal portion forms a loop (36A, 36B), the fixing member (40A, 40B) passing through said loop (36A, 36B) in said locked position so as to impede relative translation movement between the proximal portion (32A, 32B) and the spacer (10), in the first direction (D1A, D1B).

2. An intervertebral device according to claim 1, wherein the second hole (22A, 22B) extends in a second direction (D2A, D2B) which is substantially orthogonal to the first direction (D1A, D1B).

3. An intervertebral device according to claim 1 or 2, wherein the fixing member (40A, 40B) has first and second parts (42A, 42B; 46A, 46B) and an intermediate part (44A, 44B) therebetween, wherein the first and second parts (42A, 42B; 46A, 46B) are substantially straight and the intermediate part (44A, 44B) is elbow-shaped, the first part (42A, 42B) going through the second hole (22A, 22B) of the spacer and the second part (46A, 46B) resting on an outer face of the spacer when the fixing member (40A, 40B) is in its locked position.

4. An intervertebral device according to claim 3, wherein the spacer (10) is provided with a notch (50A, 50B) on its outer face, this notch extending from the second hole (22A, 22B) in a third direction (D3A, D3B) which is substantially orthogonal to the second direction (D2A, D2B), and being adapted to receive the second part (46A, 46B) of the fixing member (40A, 40B).

5. An intervertebral device according to claim 4, wherein said notch (50A, 50B) extends in a third direction (D3A, D3B) which is substantially orthogonal to the first direction (D1A, D1B).

6. An intervertebral device according to claim 4 or 5, wherein the spacer (10) comprises at least one finger or lip (52A, 52B) extending over said notch (50A, 50B) and being adapted to retain the second part (46A, 46B) of the fixing member (40A, 40B) in said notch.

7. An intervertebral device according to any one of claims 1 to 6, wherein the spacer (10) further comprises at least one fixing system (60A, 60B) for fixing a distal portion (34A, 34B) of the elongated member (30A, 30B) to the spacer (10).

8. An intervertebral device according to any one of claims 1 to 7, wherein at least one of said two opposite faces (12A, 12B) comprises a groove (14A, 14B) defined between two flanges (16A, 16B; 18A, 18B), the first hole (20A, 20B) going through one of said flanges (16A, 16B) and opening into the face (12A, 12B) of the spacer (10).

9. An intervertebral device according to claim 8, wherein the second hole (22A, 22B) opens into a side face of the spacer (10).

## Patentansprüche

1. Zwischenwirbelvorrichtung, welche zwischen zwei Wirbelfortsätzen zweier Wirbel anzuordnen ist, umfassend:
einen Abstandshalter (10), welcher zwei gegenüberliegende Flächen (12A, 12B) aufweist, wobei jede Fläche dazu ausgelegt ist, mit einem Wirbelfortsatz (SP1, SP2) gekoppelt zu werden, und
wenigstens ein langgestrecktes Element (30A, 30B) zum Beibehalten der Kopplung der Flächen (12A, 12B) mit den Wirbelfortsätzen (SP1, SP2), wobei das langgestreckte Element einen proximalen Abschnitt (32A, 32B) aufweist,
wobei der Abstandshalter (10) mit wenigstens einem Satz von ersten und zweiten Öffnungen (20A, 20B; 22A, 22B) versehen ist, wobei die erste Öffnung (20A, 20B) dazu ausgelegt ist, den proximalen Abschnitt (32A, 32B) aufzunehmen, welcher in die erste Öffnung in einer ersten Richtung (D1A, D1B) einzuführen ist, und
wobei die Vorrichtung (1) ferner ein Befestigungselement (40A, 40B) umfaßt, das zwischen einer nicht verriegelten Stellung und einer verriegelten Stellung beweglich ist,
wobei die Zwischenwirbelvorrichtung **dadurch gekennzeichnet ist, daß**
die zweite Öffnung (22A, 22B) die erste Öffnung kreuzt,
das Befestigungselement (40A, 40B) durch die zweite Öffnung (22A, 22B) geht und in der verriegelten Stellung in die erste Öffnung (20A, 20B) vorsteht, und
der proximale Abschnitt eine Schlaufe (36A, 36B) bildet, wobei das Befestigungselement (40A, 40B) in der verriegelten Stellung durch die Schlaufe (36A, 36B) verläuft, so daß es eine relative Translationsbewegung zwischen dem proximalen Abschnitt (32A, 32B) und dem Abstandshalter (10) in der ersten Richtung (D1A, D1B) hemmt.

2. Zwischenwirbelvorrichtung nach Anspruch 1, wobei die zweite Öffnung (22A, 22B) sich in einer zweiten Richtung (D2A, D2B) erstreckt, welche im wesentlichen orthogonal zu der ersten Richtung (D1A, D1B) ist.

3. Zwischenwirbelvorrichtung nach Anspruch 1 oder 2, wobei das Befestigungselement (40A, 40B) erste und zweite Teile (42A, 42B; 46A, 46B) und einen Zwischenteil (44A, 44B) dazwischen aufweist, wobei der erste und zweite Teil (42A, 42B; 46A, 46B) im wesentlichen gerade und der Zwischenteil (44A, 44B) ellenbogenförmig ist, wobei der erste Teil (42A, 42B) durch die zweite Öffnung (22A, 22B) des Abstandshalters verläuft und der zweite Teil (46A, 46B) an einer Außenfläche des Abstandshalters verbleibt, wenn das Befestigungselement (40A, 40B) in der verriegelten Stellung ist.

4. Zwischenwirbelvorrichtung nach Anspruch 3, wobei der Abstandshalter (10) auf seiner Außenfläche mit einer Aussparung (50A, 50B) versehen ist, wobei sich diese Aussparung von der ersten Öffnung (22A, 22B) in einer dritten Richtung (D3A, D3B), die im wesentlichen orthogonal zur zweiten Richtung (D2A, D2B) verläuft, erstreckt und dazu ausgelegt ist, den zweiten Teil (46A, 46B) des Befestigungselementes (40A, 40B) aufzunehmen.

5. Zwischenwirbelvorrichtung nach Anspruch 4, wobei sich die Aussparung (50A, 50B) in einer dritten Richtung (D3A, D3B) erstreckt, welche im wesentlichen orthogonal zu der ersten Richtung (D1A, D1B) ist.

6. Zwischenwirbelvorrichtung nach Anspruch 4 oder 5, wobei der Abstandshalter (10) wenigstens einen Finger oder eine Lippe (52A, 52B) umfaßt, der/die sich über die Aussparung (50A, 50B) erstreckt und dazu ausgelegt ist, den zweiten Teil (46A, 46B) des Befestigungselementes (40A, 40B) in der Aussparung zu halten.

7. Zwischenwirbelvorrichtung nach einem der Ansprüche 1 bis 6, wobei der Abstandshalter (10) ferner wenigstens ein Befestigungssystem (60A, 60B) zum Befestigen eines distalen Abschnittes (34A, 34B) des langgestreckten Elements (30A, 30B) an dem Abstandshalter (10) umfaßt.

8. Zwischenwirbelvorrichtung nach einem der Ansprüche 1 bis 7, wobei wenigstens eine der beiden gegenüberliegenden Flächen (12A, 12B) eine Nut (14A, 14B) umfaßt, welche zwischen zwei Flanschen (16A, 16B; 18A, 18B) definiert ist, wobei die erste Öffnung (20A, 20B) durch einen der Flansche (16A, 16B) verläuft und in der Fläche (12A, 12B) des Abstandshalters (10) mündet.

9. Zwischenwirbelvorrichtung nach Anspruch 8, wobei die zweite Öffnung (22A, 22B) in eine Seitenfläche des Abstandshalters (10) mündet.

## Revendications

1. Dispositif intervertébral à placer entre deux apophyses épineuses de deux vertèbres, comprenant :
une cale (10) ayant deux faces opposées (12A, 12B), chaque face étant adaptée pour engager une apophyse épineuse (SP1, SP2), et
au moins un élément allongé (30A, 30B) destiné à maintenir l'engagement des faces (12A, 12B) sur les apophyses épineuses (SP1, SP2), ledit élément allongé ayant une partie proximale (32A, 32B),
la cale (10) étant pourvue d'au moins un ensemble de premier et deuxième trous (20A, 20B ; 22A, 22B), le premier trou (20A, 20B) étant adapté pour recevoir ladite partie proximale (32A, 32B) qui doit être insérée dans le premier trou dans une première direction (D1A, D1B), et
le dispositif (1) comprenant en outre un élément de fixation (40A, 40B) mobile entre une position déverrouillée et une position verrouillée,
le dispositif intervertébral étant **caractérisé en ce que**
le deuxième trou (22A, 22B) intersecte le premier trou,
l'élément de fixation (40A, 40B) passe à travers le deuxième trou (22A, 22B) et dépasse dans le premier trou (20A, 20B) dans ladite position verrouillée, et
ladite partie proximale forme une boucle (36A, 36B), l'élément de fixation (40A, 40B) passant à travers ladite boucle (36A, 36B) dans ladite position verrouillée de façon à entraver un mouvement de translation relatif entre la partie proximale (32A, 32B) et la cale (10), dans la première direction (D1A, D1B).

2. Dispositif intervertébral selon la revendication 1, dans lequel le deuxième trou (22A, 22B) s'étend dans une deuxième direction (D2A, D2B) qui est sensiblement perpendiculaire à la première direction (D1A, D1B).

3. Dispositif intervertébral selon la revendication 1 ou 2, dans lequel l'élément de fixation (40A, 40B) a des première et deuxième parties (42A, 42B ; 46A, 46B) et une partie intermédiaire (44A, 446) entre elles, les première et deuxième parties (42A, 42B ; 46A, 46B) étant sensiblement droites et la partie intermédiaire (44A, 44B) étant coudée, la première partie (42A, 42B) passant à travers le deuxième trou (22A, 22B) de la cale et la deuxième partie (46A, 46B) reposant sur une face externe de la cale quand l'élément de fixation (40A, 40B) est dans sa position verrouillée.

4. Dispositif intervertébral selon la revendication 3, dans lequel l'cale (10) est pourvue d'une encoche (50A, 50B) sur sa face externe, cette encoche s'étendant depuis le deuxième trou (22A, 22B) dans une troisième direction (D3A, D3B) qui est sensiblement perpendiculaire à la deuxième direction (D2A, D2B), et étant adaptée pour recevoir la deuxième partie (46A, 46B) de l'élément de fixation (40A, 40B).

5. Dispositif intervertébral selon la revendication 4, dans lequel ladite encoche (50A, 50B) s'étend dans une troisième direction (D3A, D3B) qui est sensiblement perpendiculaire à la première direction (D1A, D1B).

6. Dispositif intervertébral selon la revendication 4 ou 5, dans lequel l'cale (10) comprend au moins un doigt ou une lèvre (52A, 52B) s'étendant au-dessus de ladite encoche (50A, 50B) et qui est adapté(e) pour retenir la deuxième partie (46A, 46B) de l'élément de fixation (40A, 40B) dans ladite encoche.

7. Dispositif intervertébral selon l'une quelconque des revendications 1 à 6, dans lequel la cale (10) comporte en outre au moins un système de fixation (60A, 60B) pour fixer une partie distale (34A, 34B) de l'élément allongé (30A, 30B) à la cale (10).

8. Dispositif intervertébral selon l'une quelconque des revendications 1 à 7, dans lequel au moins une desdites deux faces opposés (12A, 12B) comprend une rainure (14A, 14B) définie entre deux rebords (16A, 166 ; 18A, 18B), le premier trou (20A, 20B) passant à travers un desdits rebords (16A, 16B) et s'ouvrant dans la face (12A, 12B) de la cale (10).

9. Dispositif intervertébral selon la revendication 8, dans lequel le deuxième trou (22A, 22B) débouche sur une face latérale de la cale (10).
